# EUROPÄISCHE PATENTSCHRIFT

(11) **EP 3 689 230 B1**
(45) Veröffentlichungstag und Bekanntmachung des Hinweises auf die Patenterteilung: **14.08.2024**
(21) Anmeldenummer: 19000517.3
(22) Anmeldetag: 13.11.2019
(51) Int. Cl.: A61B 5/00, A61B 5/024, A61B 5/08

(54) **MOBILISATIONSROLLSTUHL**
MOBILISATION WHEELCHAIR
FAUTEUIL ROULANT DE MOBILISATION

(30) Priorität: 31.01.2019 DE 102019000667
(43) Veröffentlichungstag der Anmeldung: 05.08.2020
(73) Patentinhaber: Reha & Medi Hoffmann GmbH, 04329 Leipzig (DE)
(72) Erfinder: Hoffmann, Winfried, 04683 Naunhof (DE); Hoffmann, Falk, 04684 Naunhof (DE)
(74) Vertreter: Weidner Stern Jeschke

(56) Entgegenhaltungen:
- DE-A1- 102015 001 846
- US-A1- 2011 034 302
- US-A1- 2015 216 451
- US-A1- 2017 136 295
- US-A1- 2017 172 827

## Beschreibung

Die Erfindung betrifft einen Mobilisationsrollstuhl, wobei der Mobilisationsrollstuhl eine Positioniereinrichtung mit einem elektromotorischen und steuerbaren/regelbaren Antrieb besitzt.

Aus der EP 2 227 210 B1 ist ein Patientenbett zur langfristigen Lagerung eines Patienten mit integrierter und kombinierter Überwachungs- und Therapieeinrichtung zur Steuerung physiologischer Parameter bei bettlägerigen Patienten in der Akut- und Rehabilitationsphase und zur Mobilisierung des Patienten bekannt. Dieses Patientenbett kann motorisch bezüglich des Neigungswinkels der Liegefläche mittels eines ansteuerbaren Antriebs verändert bzw. geregelt werden. Die Regelung zur Ansteuerung dieses Antriebs erfolgt in Abhängigkeit wenigsten eines gemessenen physiologischen Parameters des Patienten hin zu einem Sollwert. Das Patientenbett besitzt eine Einrichtung zur Bein- und/oder Fußmobilisierung, wobei die Intensität der Mobilisierung in Abhängigkeit wenigsten des einen gemessenen physiologischen Parameters des Patienten, nämlich Blutdruck, Herzfrequenz, Hirndruck, Körpertemperatur, Blutzucker, Schweißregulation, Puls, Grundumsatz, geregelt wird. Die Regelung und Steuerung erfolgen über eine entsprechende Hard- und Software. Die Mess- und Monitoreinheit erfasst und dokumentiert kontinuierlich Herzfrequenz und Blutdruck, sowie ggf. den Hirndruck. Dabei wird der gemessene IST-Wert mit einem Soll-Wert bzw. - Bereich abgeglichen und in Abhängigkeit der Abweichung wird die Neigung des Patienten verändert, um in den Soll-Bereich zu gelangen.

Ziel dieser Überwachungs- und Therapieeinrichtung ist die Aufrechterhaltung der Homöostase des Patienten.

Aus der DE 10 2015 001 846 B4 ist ein Krankenfahrstuhl mit Vibrationseinheit bekannt, wobei die Vibrationseinheit horizontale Schwingungen oder zur jeweiligen Körperoberfläche des Patienten parallelverlaufende Schwingungen im Bereich von 1 bis 100 Hz erzeugt und die Vibrationseinheit im Bereich der Fußstütze, des Sitzes und/oder der Rückenlehne des Krankenfahrstuhls angeordnet ist oder sind. Durch die horizontale Schwingungsebene oder zur jeweiligen Körperoberfläche des Patienten parallelverlaufende Schwingungen werden die Belastungen auf die Gelenke und das Skelett vermieden, die bei anders gerichteten Schwingungsvektoren als stark belastend bekannt sind. Dieser Krankenfahrstuhl bzw. Mobilisationsrollstuhl wird u.a. im Rahmen der Erst- und Frühmobilisation und physiotherapeutischer Behandlungen erfolgreich einsetzt.

Aus der US 2017/0172827 A1 ist ein Patientenunterstützungssystem mit einem Stuhl und einer Patientenunterstützungsfläche bekannt, bei dem mittels einer Steuerung eine Betriebsgeschwindigkeit von Aktuatoren für den Sitz, die Rücklehne und die Beinlehne der Patientenunterstützungsfläche und/oder einer Patientenhebevorrichtung in Reaktion auf ein Benutzereingabegerät oder ein Erfassungssystem mit Patientenzustandssensoren eingestellt wird.

Die US 2015/216451 A1 betrifft eine Personenstützvorrichtung in Form eines Krankenbettes mit einer sensorischen Steuerung für die Lagerung und Überwachung einer Person. Das Steuersystem umfasst mehrere Sensoren und Steuermodule, mit denen die Lageposition und/oder die Anwesenheit des Patienten überwacht und erfasst wird.

Dabei besteht bei Verwendung eines solchen Mobilisationsrollstuhls, aber auch anderer, seit längerem ein Bedarf diese physiotherapeutischen Behandlungen für den Genesungsprozess des Patienten und das behandelnde medizinische Personal effektiver zu gestalten und den Verlauf der Behandlung computerbasiert und sicher zu dokumentieren.

Mit dem Gesetz zur Verbesserung der Rechte von Patientinnen und Patienten vom 20.02.2013 werden beispielsweise die Pflichten der Behandelnden, u.a. zur Dokumentation der Behandlung, in Deutschland geregelt. Es ist diesbezüglich eine Verlaufsdokumentation zu führen und kontinuierlich je Behandlungseinheit fortzuschreiben. Diese soll die im Einzelnen erbrachten Leistungen, die Reaktion des Patienten, ggf. Besonderheiten bei der Durchführung, und die Kennzeichnung bzw. Zuordnung des jeweils Behandelnden ermöglichen.

Mit der abschließenden Therapiedokumentation werden insbesondere der Problem- und Behandlungsverlauf nachvollziehbar, der Soll-IST Vergleich der Therapieziele sowie eine prognostische Einschätzung möglich.

Eine solche Dokumentation ist eine zusätzliche administrative Aufgabe, die neben der eigentlichen Tätigkeit des Therapeuten zu erbringen ist. Die Erstellung der kontinuierlichen Verlaufsdokumentation und der abschließenden Therapiedokumentation beanspruchen bis zu einem Viertel der Arbeitszeit des Therapeuten. Zudem gibt es für Physiotherapeuten bisher keine standardisierten Dokumentationsrichtlinien.

Aufgabe der Erfindung ist es, einen Mobilisationsrollstuhl bereit zu stellen, welcher eine Interaktion zwischen Mobilisationsrollstuhl und zu behandelnden Patienten ermöglicht, um eine Optimierung der Behandlung zu ermöglichen und es zu verhindern, Belastungsgrenzen des Patienten, insbesondere Grenzwerte von Vitaldaten, zu überschreiten. Diese Optimierung der Behandlung soll ein an- und abschwellendes Belastungsszenario innerhalb vorgegebener Grenzwerte durch die Positionsverstellung des Mobilisationsrollstuhls und physiotherapeutische Maßnahmen als Option einschließen. Dies soll auch ohne ein direktes Eingreifen des Therapeuten ermöglicht sein.

Die vorliegende Erfindung hat somit auch zum Ziel, die Effizienz physiotherapeutischer Behandlungen zu verbessern. Die Therapeuten sollen vom erforderlichen Dokumentationsaufwand möglichst entlastet werden, um sich ihrer eigentlichen hochqualifizierten Behandlungsaufgabe widmen zu können. Es soll außerdem ermöglicht sein, eine Verlaufsdokumentation automatisch zu erstellen und dadurch die Therapeuten von administrativen Aufgaben zu entlasten. Die Therapeuten werden somit stärker entsprechend ihrer Qualifikation und damit wirtschaftlicher eingesetzt.

Einhergehend soll der Therapieverlauf transparenter, genauer, manipulationssicher, komplexer und maschinell auswertbar aufgezeichnet werden. Damit soll u.a. die Voraussetzung geschaffen werden, um ein interaktives Agieren zu ermöglichen, um die Patientensicherheit zu erhöhen.

Der Mobilisationsrollstuhl soll außerdem für schwer betroffene Patienten ohne posturale Kontrolle geeignet sein.

Die Aufgabe der Erfindung wird durch einen Mobilisationsrollstuhl mit den Merkmalen gemäß Anspruch 1 gelöst.

Erfindungswesentlich ist, dass der Mobilisationsrollstuhl 7 zumindest eine Messeinheit 13, eine Messwerterfassungs- und Messwertverarbeitungseinheit 14, einen Datenspeicher 15, eine mikroprozessorbasierte Datenverarbeitungseinheit 16, eine Anzeige- und Eingabeeinheit 17 und eine Steuer- und Regeleinheit 6 besitzt, welche kommunizierend miteinander verbunden sind.

Die Messeinheit 13, die Messwerterfassungs- und Messwertverarbeitungseinheit 14, der Datenspeicher 15, die mikroprozessorbasierte Datenverarbeitungseinheit 16, die Anzeige- und Eingabeeinheit 17 und die Steuer- und Regeleinheit 6 sind in technisch bekannter Art und Weise ausgewählt und konfiguriert, so dass Mess- und Steuerdaten in üblicher Art und Weise übertragen, gespeichert und verarbeitet werden können, d.h. diese Baugruppen sind in bekannter Art und Weise kommunizierend miteinander verbunden.

Eine Messeinheit 13 im Sinne der Erfindung ist eine übliche elektrisch betriebene Einheit zur Messung von Vitaldaten, welche zumindest eine Datenschnittstelle zum Auslesen und/oder Übertragen von gemessenen Vitaldaten besitzt. Diese kommuniziert, beispielsweise drahtlos, in üblicher Art und Weise zumindest mit der Messwerterfassungs- und Messwertverarbeitungseinheit 14.

Auf Basis ausgewählter Daten wird somit eine Therapie optimierbar. Dabei wird auch auf Basis von Daten von vorherigen Therapiesitzungen und der aktuellen ein optimales dynamisches Training realisiert, welches durch eine ansteigende Belastbarkeit und Konstitution charakterisiert ist. Die jeweilige Tagesform und andere Einflussfaktoren auf den Gesundheitszustand des Patienten können damit erstmals einbezogen werden.

Die Genauigkeit kann entsprechend der medizinischen Erfordernisse nahezu beliebig mittels der Abtastrate bzw. Taktfrequenz der Aufzeichnungen eingestellt werden.

Es lassen sich eine Vielzahl zusätzlicher Vitalparameter erfassen und in einem komplexen Zusammenhang zum Therapieverlauf darstellen.

Schließlich werden durch die quantitative Darstellung des Verlaufs in digitalisierter Form sowohl historische als auch vergleichende maschinelle Auswertungen möglich.

Die vom physiotherapeutischen Mobilisationsrollstuhl 7 elektronisch abgetasteten Positionen, in denen sich der Patient befindet, seine durchgeführten Übungen, seine dabei aufgebrachten Kräfte und die Intensität äußerer Reize, beispielsweise durch Vibration des Fußtritts, werden kontinuierlich mit den Vitalparametern, beispielsweise den Puls- und Atemfrequenzen, verglichen. Sofern diese Vitalparameter ärztlich vorgegebene Grenzwerte erreichen, wird der behandelnde Therapeut alarmiert und/oder es erfolgt eine automatische Interaktion zwischen dem Mobilisationsrollstuhl 7 und dem Patienten.

In Abhängigkeit von Voreinstellungen kann die Therapie unterbrochen oder der Mobilisationsrollstuhl 7 in eine gewünschte Position automatisch gesteuert werden. Diese Biofeedback-Funktionalität lässt sich schließlich dazu nutzen, durch Rückkopplung auf die Steuerung des Mobilisationsrollstuhls 7 die Vitalparameter des Patienten über einen bestimmten Zeitraum, möglicherweise mit dynamischem Verlauf in einem vorgegebenen Bereich zu halten.

Die abhängigen Ansprüche 2 bis 7 enthalten vorteilhafte Ausgestaltungen der Erfindung ohne diese damit zu begrenzen.

Bevorzugt ist, dass der Mobilisationsrollstuhl 7 eine Alarmierungseinrichtung 4, bevorzugt eine akustische und/oder optische, besitzt, welche bei Erreichen zumindest eines Grenzwertes (Soll-Wertes) eines Vitalwertes durch die Datenverarbeitungseinheit 16 aktiviert wird.

Diese Alarmierung kann automatisch, d.h. ohne ein direktes Eingreifen des Therapeuten, ausgelöst werden. Diese Alarmierung ist das Ergebnis einer Interaktion zwischen dem Mobilisationsrollstuhl 7 und dem Patienten nach Überschreiten des Soll-Wertes durch den Wert des IST-Wertes zumindest einen der Vitaldaten.

Durch permanente Überwachung der Vitaldaten wird die Patientensicherheit erhöht. Über eine übliche akustische und/oder optische Alarmierung bei Erreichen von Grenzwerten hinausgehend kann der Mobilisationsrollstuhl 7 für selbständige Reaktionen eingestellt werden. Eine Alarmierung kann alternativ bei Erreichen von Grenzwerten der Vitaldaten und voreingestellte Reaktion des Mobilisationsrollstuhls 7 erfolgen. Bevorzugt ist, dass zumindest die Messwerterfassungs- und Messwertverarbeitungseinheit 14, der Datenspeicher 15, die mikroprozessorbasierte Datenverarbeitungseinheit 16, die Anzeige- und Eingabeeinheit 17 und die Steuer- und Regeleinheit 6 in einem Beistellmodul 3 oder einer tragbaren Baueinheit, bevorzugt einem Tablet-PC, angeordnet sind, welches am Mobilisationsrollstuhl 7 anordenbar und mit diesem in üblicher Art und Weise kommunikationstechnisch verbindbar ist.

Im Beistellmodul 3 kann die Messeinheit 13 angeordnet und in üblicher Art und Weise kommunizierend verbunden sein.

Die Messeinheit 13 kann einen Sensor oder mehreren Sensoren zur Messung von Vitaldaten besitzen.

Damit ist in einfacher Art und Weise die Möglichkeit eröffnet, diesbezügliche Krankenfahrstühle und/oder Mobilisationsrollstühle 7 in ihrer Funktionalität zu erweitern oder nachzurüsten.

Außerdem kann ein Beistellmodul 3 gleichzeitig oder nacheinander für mehrere Mobilisationsrollstühle 7 verwendet werden.

Bei der tragbaren Baueinheit, beispielsweise einem Tablet-PC, ist die Messeinheit 13, regelmäßig nicht in dieser tragbaren Baueinheit enthalten.

Bevorzugt ist, dass aus dem Datenspeicher 15 patientenbezogene Therapiedokumentationen abgreifbar sind.

Der Mobilisationsrollstuhl 7 ist geeignet, die Rehabilitation des Patienten zu optimieren. Gezieltes Training ohne den Patienten zu überlasten oder zu unterfordern führt zu schneller Genesung bei verringertem Komplikationsrisiko.

Vorgegebene Belastungsgrenzen oder auch Trainingsszenarien lassen sich als Parameter über die Anzeige- und Eingabeeinheit 17 in das System eingeben.

Das System überwacht sodann die Einhaltung dieser Parameter der Vitaldaten durch Nachregelung der Positionen des Mobilisationsrollstuhls 7. Beispielsweise wird eine vorgegebene und in das System als Parameter eingetragene Pulsfrequenz des Patienten weitgehend konstant gehalten, indem bei abfallender Pulsfrequenz der Patient weiter aufgerichtet wird oder bei ansteigender Pulsfrequenz der Mobilisationsrollstuhl 7 sich in Richtung einer entspannenden Ruheposition verstellt. In diese Biofeedback-Funktionalität lässt sich die Überwachung weiterer Vitaldaten wie Atemfrequenz, Blutdruck oder andere einbeziehen.

Aus den im Datenspeicher 15 abgespeicherten Werten wird ersichtlich, wie lange sich der Patient in welcher Position befunden hat. Diese Grundfunktionalität wird mit einer vor Therapiebeginn einmalig manuell über die Anzeige- und Eingabeeinheit 17 eingegebenen Patienten- und Therapeutenidentifikation abgeschlossen. Weitere Daten, wie Befund, Therapieziele, Behandlungsplan und Notizen zu Besonderheiten während der Behandlung, können manuell oder über eine übliche Datenschnittstelle, hinzugefügt werden.

Aus mehreren Verlaufsdokumentationen eines Patienten wird die Therapiedokumentation zusammengefasst, die ebenso mit prognostischer Einschätzung und weiteren Notizen manuell ergänzt werden kann. Die so entstandenen Verlaufsdokumentationen und die abschließende Therapiedokumentation genügen ohne weiteren Aufwand den einschlägigen Vorgaben zur Dokumentation der Behandlung.

Die Verlaufs- und die Therapiedokumentation können über geeignete Schnittstellen als csv-File, als PDF-Dokument oder direkt in das Krankenhausinformationssystem (KIS) exportiert werden. Das Ziel, die Therapeuten vom Dokumentationsaufwand zu entlasten, wird damit grundsätzlich erreicht.

Die beschriebene Grundfunktionalität ist erweiterbar mit der automatisch ermittelten Raumtemperatur und/oder Luftfeuchtigkeit bzw. klimatischer Einflüsse am Einsatzort sowie durch Überlagerung mit zur Verfügung stehenden Vitaldaten.

Diese Vitaldaten, wie beispielsweise Atemfrequenz oder Pulsfrequenz, können sowohl durch in den Mobilisationsrollstuhl 7 eingebaute Sensoren intern gewonnen werden als auch aus externen Quellen importiert werden, wie z.B. Blutdruck, Sauerstoffsättigung, intrakranieller Druck.

Aus der Überlagerung der Daten und der computergestützten Auswertung wird erkennbar, wie beispielsweise der Blutkreislauf des Patienten auf Lageänderungen anspricht.

Die Intensität eines auf den Patienten wirkenden Vibrationsreizes, dessen Dauer und Frequenz können an der Anzeige- und Bedieneinheit des Mobilisationsrollstuhls 7 angezeigt oder eingegeben werden. Diese Intensität des Vibrationsreizes lässt sich ebenso in üblicher Art und Weise in Relation zu den Vitaldaten des Patienten darstellen.

Ein Ziel der Behandlung ist es außerdem, die Patienten möglichst aktiv in die therapeutischen Maßnahmen einzubeziehen. Zu diesem Zweck kann quer vor den Patienten eine Griffstange angebracht werden.

Jede Änderung der Position, z.B. durch Betätigen der Handbedienung vom Mobilisationsrollstuhl 7, führt zu einer Aktualisierung der grafischen Anzeige und zu einem neuen Speichereintrag.

Die grafische Darstellung der Positionen der Patientenauflagefläche ermöglicht es dem Behandelnden die Therapiesituation mit den vorgegebenen Therapiezielen und mit historischen Werten zu vergleichen.

Durch permanente Überwachung der Vitaldaten wird die Patientensicherheit erhöht. Über eine übliche akustische und/oder optische Alarmierung kann bei Erreichen von Grenzwerten hinausgehend der Mobilisationsrollstuhl 7 für selbständige Reaktionen eingestellt werden.

Wird beispielsweise eine zu hohe Pulsfrequenz beim Aufrichten des Patienten in die Stehposition erkannt/detektiert, lassen sich das Verlangsamen bzw. Unterbrechen der Bewegung und bei anhaltend hoher Pulsfrequenz der Übergang in eine schonende Entspannungsposition programmieren.

Diese Interaktion vom therapeutischem Hilfsmittel Mobilisationsrollstuhl 7 und Patient nach vorgegebenen Leitlinien unterstützt den Behandelnden in seiner Reaktion auf Besonderheiten während der Therapie.

Die jeweiligen Reaktionen des Mobilisationsrollstuhls 7 auf die Veränderung der Vitaldaten werden zuvor entsprechend der medizinischen Leitlinien in das System programmiert. Die patientenbezogenen Grenzwerte werden über die Anzeige- und Eingabeeinheit 17 als Parameter eingegeben.

Bei Ergänzung der vorgegebenen Belastungsparameter mit einer zeitlichen Komponente ergibt sich für den Patienten ein Trainingsszenario.

Beispielsweise kann nach einer stärkeren Belastung (höhere Pulsfrequenz) eine Erholungsphase mit niedrigerer Pulsfrequenz vorgesehen sein.

Zur Nachregelung kann die Positionen des Mobilisationsrollstuhl 7, aber auch die Vibrationsstärke (Frequenz und Dauer) einer Vibrationseinheit 1, in diesem Ausführungsbeispiel nicht enthalten, zur Verfügung stehen.

Weitere Merkmale, Eigenschaften und Vorteile der vorliegenden Erfindung ergeben sich aus der nachfolgenden Beschreibung von Ausführungsbeispielen unter Bezugnahme auf die Figur 1 bis 3.

Es zeigen:
- Fig. 1: eine Ausführungsform eines Mobilisationsrollstuhls 7 mit zumindest einer Vibrationseinheit 1 in einer schematischen perspektivischen Ansicht,
- Fig. 2: eine schematische Darstellung der Steuer-, Mess-, Eingabe- und Ausgabedatenströme des Mobilisationsrollstuhls 7 als Blockschaltbild,
- Fig 3: eine schematische Darstellung eines Beistellmoduls 3, und
- Fig 4: eine Ausführungsform eines Mobilisationsrollstuhls 7 mit zumindest einer Griffstange in einer schematischen perspektivischen Ansicht.

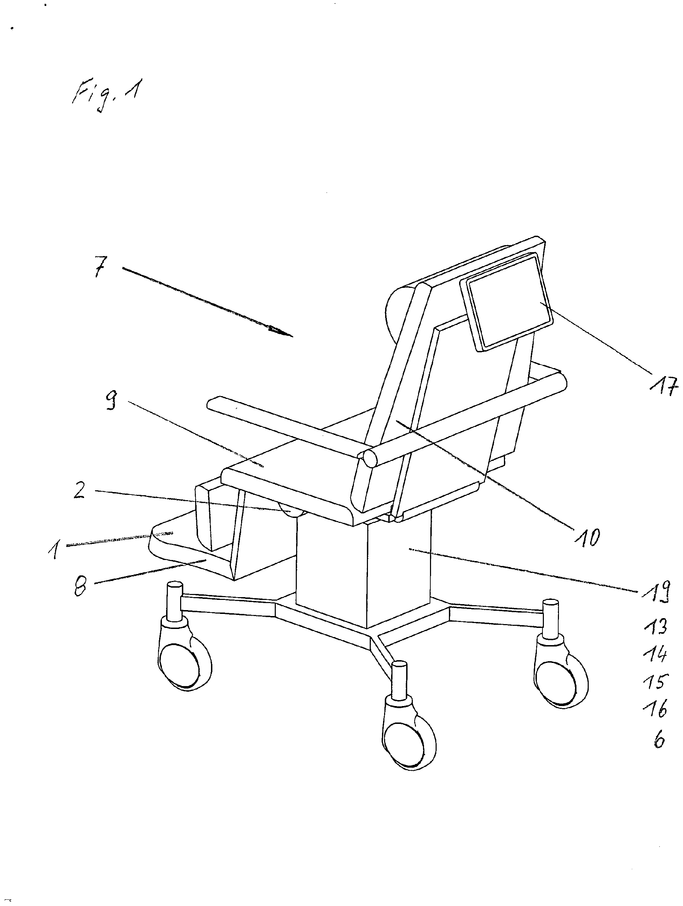

Fig. 1 zeigt eine Ausführungsform eines Mobilisationsrollstuhls 7 mit zumindest einer Vibrationseinheit 1 in einer schematischen perspektivischen Ansicht.

Die Vibrationseinheit 1 kann im Bereich der Fußstütze 8, des Sitzes 9 und/oder der Rückenlehne 10 des Mobilisationsrollstuhls 7 angeordnet sein. Die Vibrationseinheit 1 besitzt einen elektromotorischen und steuerbaren/regelbaren Antrieb 12, beispielsweise einen Unwuchtmotor.

Der Mobilisationsrollstuhl 7 besitzt eine Positioniereinrichtung 2 mit einem elektromotorischen und steuerbaren/regelbaren Antrieb 11, in Fig. 1 nicht dargestellt.

Der Mobilisationsrollstuhl 7 besitzt zumindest eine Messeinheit 13, eine Messwerterfassungs- und Messwertverarbeitungseinheit 14, einen Datenspeicher 15, eine mikroprozessorbasierte Datenverarbeitungseinheit 16, eine Anzeige- und Eingabeeinheit 17 und eine Steuer- und Regeleinheit 6, wobei diese kommunizierend miteinander verbunden sind.

Der fahrbare Mobilisationsrollstuhl 7 besitzt zumindest eine Fußstütze 8, einen Sitz 9 und eine Rückenlehne 10. Ansonsten kann der Mobilisationsrollstuhl 7 bekannte Funktionalitäten erfüllen und die diesbezüglich erforderlichen Bauteile, Baugruppen und Steuerprogramme entsprechend seinem jeweiligen Ausstattungsgrad besitzen. Diesbezüglich besitzt dieser insbesondere eine Gerätesteuerung 12, welche u.a. den elektromotorischen Antrieb 11, die Positioniereinrichtung 2 und die Vibrationseinheit 1 in üblicher Art und Weise ansteuern kann.

Die Vibrationseinheit 1 kann beispielsweise horizontale Schwingungen oder zur jeweiligen Körperoberfläche des Patienten parallelverlaufende Schwingungen im Bereich von 1 bis 100 Hz erzeugen. Der Mobilisationsrollstuhl 7 besitzt eine übliche elektrische Stromversorgungseinrichtung, in Fig. 1 nicht dargestellt, für alle elektrisch betriebenen Bauteile.

Vom Liegen, über das Sitzen bis zum Stand kann ein kontinuierlicher Bewegungsablauf durch eine Positioniereinrichtung 2, welche durch einen elektromotorischen Antrieb 11 angetrieben und durch eine Gerätesteuerung 12 in üblicher Art und Weise gesteuert wird, erfolgen, ohne dass der Patient selbst Energie aufbringen muss, um seine Position zu verändern. Dies erfolgt durch eine stufenlos veränderliche Positioniereinrichtung 2 des Mobilisationsrollstuhls7.

Die Regelung und Steuerung erfolgen über eine entsprechende in Art und Umfang übliche Hard- und Software.

Durch permanente Überwachung der Vitaldaten wird die Patientensicherheit erhöht. Über eine übliche akustische und/oder optische Alarmierung bei Erreichen von Grenzwerten hinausgehend kann der Mobilisationsrollstuhl 7 für selbständige Reaktionen eingestellt werden.

Der Mobilisationsrollstuhl 7 ermöglicht zumindest drei Patientenpositionen, nämlich eine Sitz-, Liege- oder Standposition. Der Patient sitzt, liegt oder steht im oder auf dem Mobilisationsrollstuhl 7, wobei er mit den Füßen regelmäßig das Fußteil 8 berührt.

Im Ausführungsbeispiel gemäß Fig. 1 ist die Vibrationseinheit 1 im Bereich der Fußstütze 8 angeordnet und in der Fußstütze 8 integriert. Die Vibrationseinheit 1 ist ansteuerbar, sodass insbesondere deren Frequenz, wie gewünscht, veränderbar ist.

Die Messeinheit 13, die Messwerterfassungs- und Messwertverarbeitungseinheit 14, der Datenspeicher 15, die mikroprozessorbasierte Datenverarbeitungseinheit 16, die Anzeige- und Eingabeeinheit 17 und die Steuer- und Regeleinheit 6 sind in der dargestellten Ausführungsform im Gehäuse 19 des Mobilisationsrollstuhls 7 angeordnet.

Alternativ sind diese in einem Beistellmodul 20, nicht dieses Ausführungsbeispiel betreffend, angeordnet, welches neben dem Mobilisationsrollstuhl 7 anordenbar und mit diesem insbesondere zur Datenkommunikation in üblicher Art und Weise verbindbar ist.

Der Mobilisationsrollstuhl 7 ist zur Erst- und Frühmobilisation, insbesondere in der neurologischen Frührehabilitation, geeignet, welche eine allmähliche und kontinuierliche Vertikalisierung aus einer Sitzposition heraus ermöglichen.

Ein solcher Mobilisationsrollstuhl 7 ist beispielsweise für schwer betroffene Patienten ohne posturalen Kontrolle geeignet.

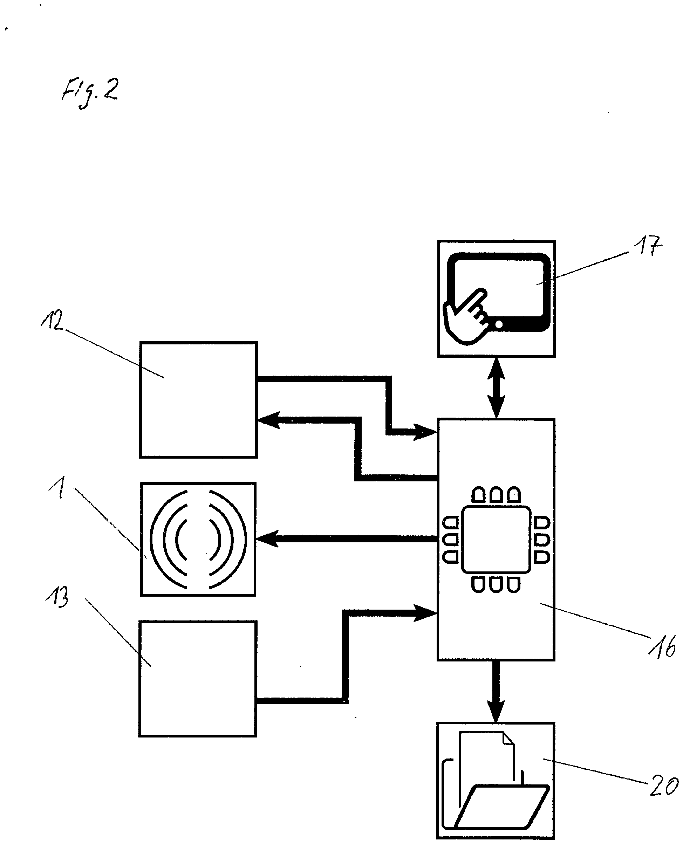

Fig. 2 zeigt eine schematische Darstellung der Steuer-, Mess-, Eingabe- und Ausgabedaten des Mobilisationsrollstuhls 7 als Blockschaltbild.

Eine Datenverarbeitungseinheit 16, welche insbesondere einen üblichen Mikroprozessor und eine diesbezügliche Software besitzt, überträgt, gespeichert und verarbeitet insbesondere Steuer-, Mess-, Eingabe- und Ausgabedaten in üblicher Art und Weise. Aus der Datenverarbeitungseinheit 16 können die Verlaufs- und die Therapiedokumentation über eine Datenausgabe 20 mit geeigneten Schnittstellen als csv-File, als PDF-Dokument oder direkt in das Krankenhausinformationssystem (KIS) exportiert werden.

Diese Datenverarbeitungseinheit 16 kann in vorgegebenen Zeitintervallen die Inkrementalsignale der Motoren aus der Steuerung des Mobilisationsrollstuhls 7 auslesen und daraus die Positionen der Patientenauflagefläche, wie Neigung der Rückenlehne 10 und der Fußstütze 8, Neigung des Sitzes 9 sowie die Höhe errechnen. Die Berechnung erfolgt in üblicher Art und Weise nach Funktionen, die der Gerätekonstruktion zugrunde liegen. Hilfsweise können auch empirisch gewonnene tabellarische Daten herangezogen werden.

Die so ermittelten Positionen der Patientenauflagefläche können auf einem Display oder Anzeige- und Eingabeeinheit 17 grafisch dargestellt und mit einem Zeitstempel versehen abgespeichert werden.

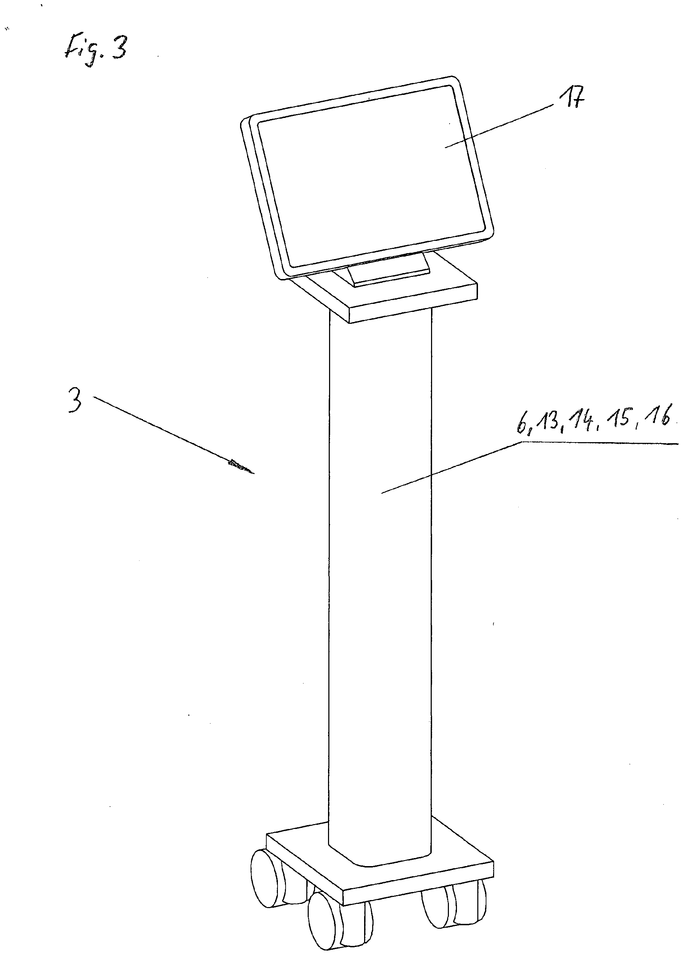

Fig. 3 zeigt eine schematische Darstellung eines verfahrbaren Beistellmoduls 3. Zumindest die Messeinheit 13, die Messwerterfassungs- und Messwertverarbeitungseinheit 14, der Datenspeicher 15, die mikroprozessorbasierte Datenverarbeitungseinheit 16, die Anzeige- und Eingabeeinheit 17 und die Steuer- und Regeleinheit 6 sind in einem Beistellmodul 3 angeordnet, welches sich neben dem Mobilisationsrollstuhl 7, in Fig. 3 nicht dargestellt, anordnen und sich mit diesem, insbesondere zur Datenkommunikation, in üblicher Art und Weise verbinden lässt.

Das Beistellmoduls 3 kann eine gesonderte Energieversorgungseinrichtung, beispielsweise eine elektrische Batterieeinheit, besitzen oder mit einer externen Energiequelle verbindbar sein.

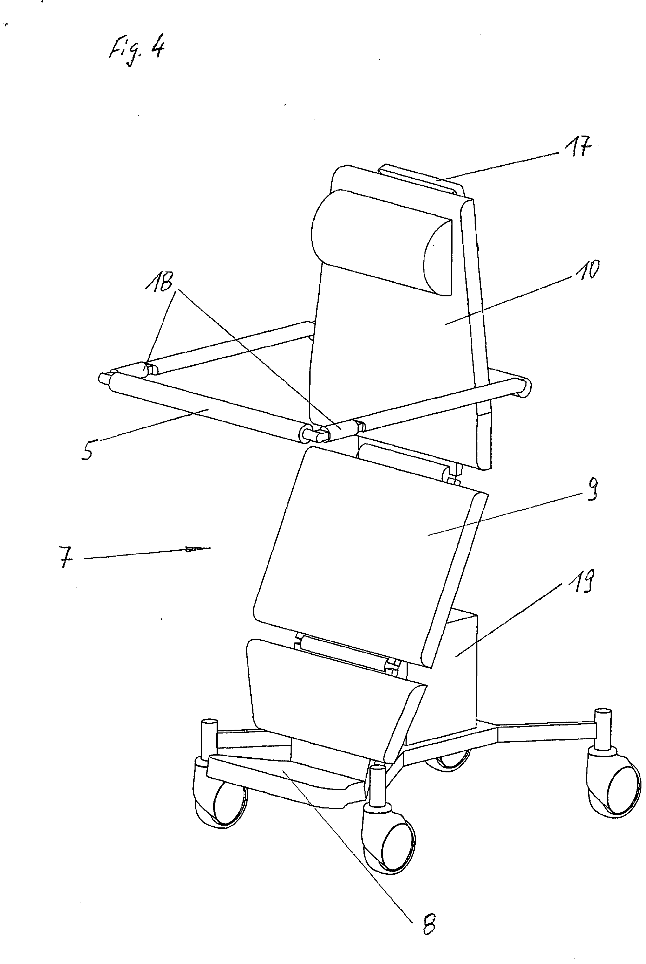

Fig. 4 zeigt eine Ausführungsform eines Mobilisationsrollstuhls 7 mit zumindest einer Griffstange 5 in einer schematischen perspektivischen Ansicht.

Der Mobilisationsrollstuhl 7 besitzt eine Positioniereinrichtung 2 mit einem elektromotorischen und steuerbaren/regelbaren Antrieb 11, in Fig. 4 nicht dargestellt. Der Mobilisationsrollstuhl 7 besitzt zumindest eine Messeinheit 13, eine Messwerterfassungs- und Messwertverarbeitungseinheit 14, einen Datenspeicher 15, eine mikroprozessorbasierte Datenverarbeitungseinheit 16, eine Anzeige- und Eingabeeinheit 17 und eine Steuer- und Regeleinheit 6, wobei diese kommunizierend miteinander verbunden sind.

Der fahrbare Mobilisationsrollstuhl 7 besitzt zumindest eine Fußstütze 8, einen Sitz 9 und eine Rückenlehne 10. Ansonsten kann der Mobilisationsrollstuhl 7 bekannte Funktionalitäten erfüllen und die diesbezüglich erforderlichen Bauteile, Baugruppen und Steuerprogramme entsprechend seinem jeweiligen Ausstattungsgrad besitzen.

Diese Griffstange 5 ist quer vor der Patientenposition, insbesondere lösbar oder verschwenkbar am Mobilisationsrollstuhls 7, angebracht. Die Griffstange 5 ist beidseitig mit Messaufnehmern 18 versehen, die die vom Patienten eingebrachte Zug- bzw. Druckkraft messen. Diese gemessenen Messdaten werden über eine übliche Datenschnittstelle an die Messwerterfassungs- und Messwertverarbeitungseinheit 14 übertragen.

In der Liegeposition kann sich der Patient an der Griffstange 5 nach oben ziehen. Insbesondere beim Übergang vom Sitzen in den Stand kann der Patient das physiologisch korrekte Aufstehen üben. Nach elektronischer Auswertung der eingebrachten Kräfte wird dem Patienten vermittels der Steuerung zur Positionierung des Mobilisationsrollstuhls 7 die individuell benötigte Unterstützung beim Aufstehen gegeben. In der Stehposition sind Übungen zur Verbesserung der Koordinationsfähigkeit möglich. Die Kräftebalance wird dem Patienten auf einem Bildschirm dargestellt, wodurch er selbst zur koordinierten Aktion motiviert wird.

Diese Griffstange 5 am Mobilisationsrollstuhl 7 eröffnet den Patienten vielfältige Ansatzpunkte zum Muskelaufbau, zur Verbesserung der Bewegungsabläufe und zum

Koordinationstraining durch eigene Aktivitäten. Somit kann ohne zusätzlichen Aufwand umfangreiches Datenmaterial in die Verlaufsdokumentation einfließen, wodurch diese wesentlich aussagefähiger wird.

### Liste der Bezugszeichen

1 Vibrationseinheit
2 Positioniereinrichtung
3 Beistellmodul
4 Alarmierungseinrichtung
5 Griffstange
6 Steuer- und Regeleinheit
7 Mobilisationsrollstuhl
8 Fußstütze
9 Sitz
10 Rückenlehne
11 Antrieb
12 Gerätesteuerung
13 Messeinheit Vitaldaten
14 Messwerterfassungs- und Messwertverarbeitungseinheit
15 Datenspeicher
16 Datenverarbeitungseinheit
17 Anzeige- und Eingabeeinheit
18 Messaufnehmern
19 Gehäuse
20 Datenausgabe

## Patentansprüche

1. Mobilisationsrollstuhl, wobei der Mobilisationsrollstuhl (7) eine Positioniereinrichtung (2) mit einem elektromotorischen und steuerbaren/regelbaren Antrieb (11) besitzt, und der Mobilisationsrollstuhl (7) zumindest eine Messeinheit (13) zum Messen von Vitalwerten, eine Messwerterfassungs- und Messwertverarbeitungseinheit (14), einen Datenspeicher (15), eine mikroprozessorbasierte Datenverarbeitungseinheit (16), eine Anzeige- und Eingabeeinheit (17) und eine Steuer- und Regeleinheit (6) besitzt, wobei diese bei kontinuierlicher Messung kommunizierend miteinander verbunden sind, **dadurch gekennzeichnet, dass** der Mobilisationsrollstuhl (7) durch die Positioniereinrichtung (2) mit dem elektromotorischen Antrieb (11) zumindest in eine Sitz-, Liege- und Standposition einstellbar ist und aus dem Datenspeicher (15) patientenbezogene Therapiedokumentationen abgreifbar sind, sodass durch eine Nachregelung der zumindest einen Sitz-, Liege- und Standposition mittels der Steuer- und Regeleinheit (6) und der Positioniereinrichtung (2) eine Einhaltung von Vitalwerten realisierbar ist.

2. Mobilisationsrollstuhl gemäß Anspruch 1, **dadurch gekennzeichnet, dass** der Mobilisationsrollstuhl (7) eine Alarmierungseinrichtung (4), bevorzugt eine akustische und/oder optische, besitzt, welche bei Erreichen zumindest eines Grenzwertes (Soll-Wertes) eines Vitalwertes durch die Datenverarbeitungseinheit (16) aktiviert wird.

3. Mobilisationsrollstuhl gemäß Anspruch 1, **dadurch gekennzeichnet, dass** im Datenspeicher (15) zumindest patientenbezogene Messdaten, wie die Vitalwerte Atemfrequenz, Pulsfrequenz und Blutdruck, und/oder Werte, wie lange sich ein Patient in welcher Position befunden hat, gespeichert werden.

4. Mobilisationsrollstuhl gemäß Anspruch 1, **dadurch gekennzeichnet, dass** zumindest die Messwerterfassungs- und Messwertverarbeitungseinheit (14), der Datenspeicher (15), die mikroprozessorbasierte Datenverarbeitungseinheit (16), die Anzeige- und Eingabeeinheit (17) und die Steuer- und Regeleinheit (6) in einer Baueinheit im Mobilisationsrollstuhl (7), einer tragbaren Baueinheit, bevorzugt einem Tablet-PC, oder einem Beistellmodul (3) angeordnet sind.

5. Mobilisationsrollstuhl gemäß Anspruch 1, **dadurch gekennzeichnet, dass** aus dem Datenspeicher (15) patientenbezogene Therapiedokumentationen abgreifbar sind, welche durch Überlagerung von Vitaldaten des Patienten aus internen oder externen Datenquellen errechnet worden sind.

6. Mobilisationsrollstuhl gemäß Anspruch 1, **dadurch gekennzeichnet, dass** die Messeinheit (13) zur Messung der vom Patienten aufgebrachten Gegenkraft und die Messwerterfassungs- und Messverarbeitungseinheit (14) zur Auswertung der Antriebsregelung bezüglich Kraft und Geschwindigkeit dient.

7. Mobilisationsrollstuhl gemäß Anspruch 1, **dadurch gekennzeichnet, dass** der Mobilisationsrollstuhl (7) zumindest eine Vibrationseinheit (1) besitzt, wobei die Vibrationseinheit (1) im Bereich der Fußstütze (8), des Sitzes (9) und/oder der Rückenlehne (10) des Krankenfahrstuhls (7) angeordnet ist und die Vibrationseinheit (1) einen elektromotorischen und steuerbaren/regelbaren Antrieb besitzt.

## Claims

1. Mobilisation wheelchair, wherein the mobilisation wheelchair (7) has a positioning device (2) with an electromotive drive (11) controllable by open-loop/closed-loop control, and the mobilisation wheelchair (7) has at least one measuring unit (13) for measuring vital signs, a measurement value detection and measurement value processing unit (14), a data store (15), a microprocessor-based data processing unit (16), a display and input unit (17) and an open-loop and closed-loop control unit (6), wherein these are connected communicatively to one another during continuous measurement, **characterised in that** the mobilisation wheelchair (7) is adjustable by the positioning device (2) with the electromotive drive (11) at least into a sitting, lying and standing position and patient-related therapy documentation can be retrieved from the data store (15), so that a compliance of vital signs is achievable by readjusting the at least one sitting, lying and standing position by means of the open-loop and closed-loop control unit (6) and the positioning device (2).

2. Mobilisation wheelchair according to claim 1, **characterised in that** the mobilisation wheelchair (7) has an alarm device (4), preferably an acoustic and/or optical one, which is activated when at least one limit value (target value) of a vital sign is reached by the data processing unit (16).

3. Mobilisation wheelchair according to claim 1, **characterised in that** at least patient-related measurement data, such as the vital signs of respiratory rate, pulse rate and blood pressure, and/or values relating to how long a patient has been in what position are stored in the data store (15).

4. Mobilisation wheelchair according to claim 1, **characterised in that** at least the measurement value detection and measurement value processing unit (14), the data store (15), the microprocessor-based data processing unit (16), the display and input unit (17) and the open-loop and closed-loop control unit (6) are disposed in a modular unit in the mobilisation wheelchair (7), a portable modular unit, preferably a tablet PC, or an add-on module (3).

5. Mobilisation wheelchair according to claim 1, **characterised in that** patient-related therapy documentation generated by superimposing vital data of the patient from internal or external data sources can be retrieved from the data store (15).

6. Mobilisation wheelchair according to claim 1, **characterised in that** the measuring unit (13) serves for measuring the counter force applied by the patient and the measurement value detection and measurement value processing unit (14) serves for evaluating the drive control in respect of force and speed.

7. Mobilisation wheelchair according to claim 1, **characterised in that** the mobilisation wheelchair (7) has at least one vibration unit (1), wherein the vibration unit (1) is disposed in the region of the footrest (8), the seat (9) and/or the backrest (10) of the wheelchair (7) and the vibration unit (1) has an electromotive drive controllable by open-loop/closed-loop control.

## Revendications

1. Fauteuil roulant de mobilisation, le fauteuil roulant de mobilisation (7) possédant un dispositif de positionnement (2) avec un entraînement (11) électromoteur et pouvant être commandé/régulé, et le fauteuil roulant de mobilisation (7) possédant au moins une unité de mesure (13) pour mesurer des valeurs vitales, une unité d'acquisition de valeurs mesurées et de traitement de valeurs mesurées (14), une mémoire de données (15), une unité de traitement de données à base de microprocesseur (16), une unité d'affichage et d'entrée (17) et une unité de commande et de régulation (6), celles-ci étant reliées entre elles de manière à communiquer en cas de mesure continue, **caractérisé en ce que** le fauteuil roulant de mobilisation (7) peut être ajusté par le dispositif de positionnement (2) avec l'entraînement électromoteur (11) au moins dans une position assise, couchée et debout et des documents thérapeutiques relatifs au patient peuvent être récupérés à partir de la mémoire de données (15), de telle sorte qu'un respect de valeurs vitales peut être réalisé par un réajustement de l'au moins une position assise, couchée et debout au moyen de l'unité de commande et de régulation (6) et du dispositif de positionnement (2).

2. Fauteuil roulant de mobilisation selon la revendication 1, **caractérisé en ce que** le fauteuil roulant de mobilisation (7) possède un dispositif d'alarme (4), de préférence acoustique et/ou optique, qui est activé par l'unité de traitement de données (16) lorsqu'au moins une valeur limite (valeur de consigne) d'une valeur vitale est atteinte.

3. Fauteuil roulant de mobilisation selon la revendication 1, **caractérisé en ce que**, dans la mémoire de données (15), au moins des données de mesure relatives au patient, telles que les valeurs vitales fréquence respiratoire, fréquence du pouls et pression sanguine, et/ou des valeurs indiquant combien de temps un patient s'est trouvé dans telle ou telle position, sont enregistrées.

4. Fauteuil roulant de mobilisation selon la revendication 1, **caractérisé en ce qu'**au moins l'unité d'acquisition de valeurs mesurées et de traitement de valeurs mesurées (14), la mémoire de données (15), l'unité de traitement de données à base de microprocesseur (16), l'unité d'affichage et d'entrée (17) et l'unité de commande et de régulation (6) sont agencées dans une unité de construction dans le fauteuil roulant de mobilisation (7), une unité de construction portable, de préférence une tablette PC, ou un module d'appoint (3).

5. Fauteuil roulant de mobilisation selon la revendication 1, **caractérisé en ce que** des documents thérapeutiques relatifs au patient qui ont été calculés par superposition de données vitales du patient provenant de sources de données internes ou externes peuvent être récupérés à partir de la mémoire de données (15).

6. Fauteuil roulant de mobilisation selon la revendication 1, **caractérisé en ce que** l'unité de mesure (13) sert à mesurer la force antagoniste appliquée par le patient et l'unité d'acquisition de valeurs mesurées et de traitement de valeurs mesurées (14) sert à évaluer la régulation de l'entraînement en termes de force et de vitesse.

7. Fauteuil roulant de mobilisation selon la revendication 1, **caractérisé en ce que** le fauteuil roulant de mobilisation (7) possède au moins une unité de vibration (1), l'unité de vibration (1) étant agencée dans la zone du repose-pieds (8), du siège (9) et/ou du dossier (10) du fauteuil roulant pour malade (7) et l'unité de vibration (1) possédant un entraînement électromoteur et pouvant être commandé/régulé.
